# EUROPÄISCHE PATENTSCHRIFT

(11) **EP 0 956 874 B1**
(45) Veröffentlichungstag und Bekanntmachung des Hinweises auf die Patenterteilung: **03.11.2004**
(21) Anmeldenummer: 99810405.3
(22) Anmeldetag: 07.05.1999
(51) Int. Cl.: A61M 5/24, A61M 5/315

(54) **Vorrichtung zur dosierten Verabreichung eines injizierbaren Produkts**
Device for the dosed application of an injectable product
Dispositif pour l'application dosée d'un produit injectable

(30) Priorität: 15.05.1998 DE 19821934
(43) Veröffentlichungstag der Anmeldung: 17.11.1999
(73) Patentinhaber: TecPharma Licensing AG, 3401 Burgdorf (CH)
(72) Erfinder: Hostettler, Peter, 3423 Ersigen (CH); Heiniger, Hanspeter, 4932 Lotzwil (CH)

(56) Entgegenhaltungen:
- EP-A- 0 298 067
- EP-A- 0 550 767
- WO-A-94/21316

## Beschreibung

Die Erfindung betrifft eine Vorrichtung zur dosierten Verabreichung eines injizierbaren Produkts. Die Vorrichtung umfaßt wenigstens ein Basisteil, ein vom Basisteil aufgenommenes Behältnis, eine Antriebseinrichtung und eine Dosiereinrichtung. Aus dem Behältnis wird durch Vorschieben wenigstens eines darin aufgenommenen Kolbens in eine Vorschubrichtung eine Produktdosis durch eine Nadel hindurch ausgeschüttet. Die Antriebseinrichtung weist wenigstens ein in das Behältnis ragendes Abtriebsglied auf, das bei Betätigung der Antriebseinrichtung den Kolben in Vorschubrichtung schiebt. Mittels der Dosiereinrichtung wird diejenige Weglänge eingestellt, die das Abtriebsglied bei einer Betätigung der Antriebseinrichtung relativ zum Basisteil in Vorschubrichtung des Kolbens verschoben wird.

Bei dem Basisteil handelt es sich vorzugsweise um ein das Behältnis, die Antriebseinrichtung und die Dosiereinrichtung umhüllendes Gehäuse. Die Vorrichtung dient zur Injektion oder Infusion einer im allgemeinen flüssigen Wirkstofflösung, vorzugsweise einer medizinisch oder kosmetisch wirksamen Flüssigkeit. Besonders bevorzugt betrifft die Erfindung eine tragbare Vorrichtung, insbesondere in der Form eines sogenannten Injektionspens.

Ein Injektionspen mit einer Doppelkammerampulle, wie die Erfindung ihn insbesondere auch betrifft, ist aus der EP 0 298 067 B1 bekannt.

Die Erfindung hat es sich zur Aufgabe gemacht, eine Vorrichtung der vorstehend beschriebenen Art zu schaffen, die möglichst einfach und kurz baut.

Diese Aufgabe wird bei einer Vorrichtung der vorstehend beschriebenen Art dadurch gelöst, daß wenigstens ein erstes Dosiermittel und wenigstens ein relativ zum ersten verstellbares zweites Dosiermittel der Dosiereinrichtung in dem Behältnis angeordnet sind und die Dosierung im Zusammenwirken des ersten und des zweiten Dosiermittels innerhalb des Behältnisses stattfindet.

Es wird nach der Erfindung der in Vorschubrichtung gesehen hinter dem Kolben befindliche Behältnisraum zur Aufnahme von Dosiermitteln der Dosiereinrichtung genutzt. Es kann Baulänge immer dann eingespart werden, wenn das Behältnis den Kolben rückwärtig zumindest ein Stück weit überragt. Dies ist insbesondere bei Mehrkammerampullen der Fall, bei denen hintereinander angeordnete Kammern durch Kolben voneinander getrennt und durch einen hinteren Kolben abgeschlossen werden. Da im allgemeinen der hintere Kolben zum Durchmischen der Kammerinhalte vor der ersten Injektion gegen den oder die vorderen Kolben geschoben wird, entsteht ein freier hinterer Behältnisraum zwangsläufig.

In einer bevorzugten Verwendung der Erfindung, nämlich bei einer Vorrichtung mit solch einer Mehrkammerampulle, wird gerade dieser unvermeidlich freie Behältnisraum zum Anordnen von Dosiermitteln der Dosiereinrichtung, vorzugsweise der kompletten Dosiereinrichtung, genutzt. Eine Reduzierung von Bauteilen ergibt sich darüberhinaus, wenn das bei solchen Vorrichtungen ansich bekannte Mischglied, das nach dem Stand der Technik lediglich die Aufgabe hat, den hinteren Kolben zum Vermischen der Kammerinhalte nach vorn zu schieben, gleichzeitig auch zum Träger wenigstens eines Dosiermittels weitergebildet wird und somit eine Komponente der Dosiereinrichtung ist.

In einem bevorzugten Ausführungsbeispiel der Erfindung sind das erste Dosiermittel an solch einem Mischglied und das zweite Dosiermittel unmittelbar am Abtriebsglied vorgesehen. In dieser konstruktiv sehr einfachen Ausführung findet die Dosierung unmittelbar zwischen den beiden zum Vorschieben des Kolbens relativ zueinander bewegten Komponen-ten der Vorrichtung statt. Das Mischglied kann als Mischstange ausgebildet sein und wäre in dieser Ausbildung von dem dann als Abtriebshülse ausgebildeten Abtriebsglied umgeben. Vorzugsweise ist jedoch das Mischglied als Mischhülse bzw. Mischrohr ausgebildet und umgibt das Abtriebsglied, wodurch ein konstruktiv besonders einfacher Aufbau der Antriebseinrichtung und der Dosiereinrichtung erhalten wird.

Die Dosiereinrichtung ist vorzugsweise in der Art einer Kulissenführung zwischen zwei in das Behältnis hineinragenden Komponenten der Dosiereinrichtung ausgebildet.

Die Kulissenführung wird vorteilhafterweise durch eine Ausnehmung im Bereich einer inneren Umfangsfläche eines Hülsenkörpers und einen eingreifenden Nocken gebildet. Die Ausnehmung ist das erste Dosiermittel und der Nocken das zweite. Besonders bevorzugt bildet die Ausnehmung einen Führungskanal für den Nocken.

Diejenige Komponente der Dosiereinrichtung, an der das erste Dosiermittel ausgebildet ist, kann auf einfache Weise dadurch hergestellt werden, daß die Ausnehmung erst durch Zusammenfügen von mehreren zylindrischen Körpern entsteht; wenigstens einer dieser Körper ist hohlzylindrisch bzw. schalenförmig. Vorzugsweise sind beide Körper als Hülsenkörper bzw. schalenförmig ausgebildet.

Eine treppenförmig verlaufende Ausnehmung wird mit zwei zylindrischen Körpern mit treppenförmigen Stirnflächen erhalten. Zwei schalenförmige Körper können in einen dritten Hülsenkörper in Vorschubrichtung gesehen hintereinander und zwischen sich die treppenförmige Ausnehmung ausbildend eingelegt sein.

Eine Dosierung mittels einer Einstellspindel wäre jedoch ebenso realisierbar. Der Spindeltrieb zum Dosieren würde durch die Komponente mit dem ersten Dosiermittel und eine zusätzliche Einstellhülse gebildet, die gleichzeitig als Geradführung für das Abtriebsglied dienen würde. Solche Dosiermechanismen sind bei Injektionspens grundsätzlich bekannt, nicht jedoch deren Anordnung im Behältnis.

In einer ersten Weiterbildung der Erfindung wird diejenige Komponente der Dosiereinrichtung, an der das erste Dosiermittel ausgebildet ist, vorzugsweise ein Mischglied, gleichzeitig als Übertragungsglied genutzt, das eine Vorschubbewegung des Abtriebsglieds auf das Behältnis überträgt. Diese Doppelfunktion kommt bei sogenannten Autoinjektionsgeräten zum Tragen, bei denen zum Einstechen der am Behältnis fest angebrachten Injektionsnadel das Behältnis dem Basisteil gegenüber bis in eine vordere Position vorgeschoben wird.

Bei dem injizierbaren Produkt handelt es sich vorzugsweise um eine flüssige Wirkstofflösung, insbesondere um eine medizinisch oder kosmetisch wirksame Wirkstofflösung, beispielsweise Insulin.

Bevorzugte Ausführungsbeispiele der Erfindung werden nachfolgend anhand von Figuren erläutert. Es zeigen:
- Fig. 1: ein Autoinjektionsgerät für eine zweifache Ausschüttung,
- Fig. 2: ein Injektionsgerät für eine viermalige Ausschüttung einer Wirkstofflösung und
- Fig. 3: ein Übertragungs- und Dosiermittel des Injektionsgeräts nach Fig. 2.

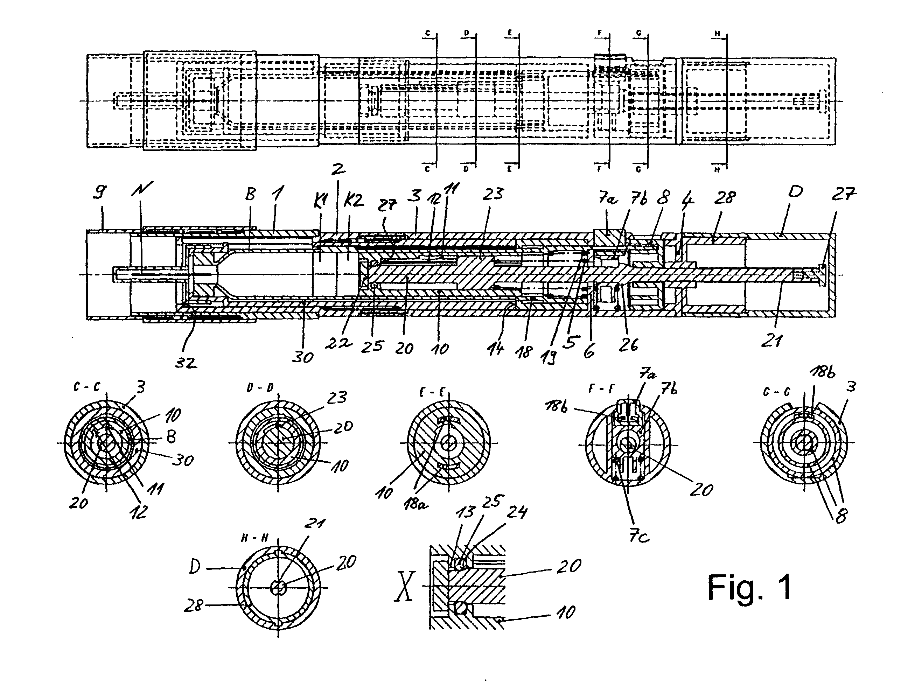

Fig. 1 zeigt im Längsschnitt und mehreren Querschnitten ein Autoinjektionsgerät in Form eines Pen mit einem eingesetzten Behältnis B, das im Ausführungsbeispiel als Doppelkammerampulle ausgebildet ist. Das Injektionsgerät ist unmittelbar nach dem Einsetzen des Behältnisses B in ein Gehäuse dargestellt, das im wesentlichen durch eine vordere Gehäusehülse 2 und eine damit verschraubte hintere Gehäusehülse 3 gebildet wird. Ein vorderes Ende der vorderen Gehäusehülse wird durch einen auf die vordere Gehäusehülse 2 aufgesteckten Nadelschutz 1 in Form einer Aufsteckhülse gebildet. An ihrer rückwärtigen Stirnseite wird die hintere Gehäusehülse 3 durch eine Gehäusekappe 4 abgedeckt.

Beim Einsetzen des Behältnisses B wird das Behältnis B in einen Behältnishalter 30 bis auf Anschlag eingeschoben, der in der vorderen Gehäusehülse 2 aufgenommen ist und aus dieser vor dem Zusammensetzen des Gerätes nach hinten vorragt. Der Behältnishalter 30 dient der Halterung und Zentrierung des Behältnisses B. Der Behältnishalter 30 ist gegen die rückstellende Kraft eines als Druckfeder ausgebildeten Rückstellelements 31 dem Gehäuse gegenüber aus seiner in Fig. 1 dargestellten hinteren Position in eine vordere Position verschiebbar. Das im Behältnishalter 30 aufgenommene Behältnis B wird dabei zusammen mit dem Behältnishalter 30 verschoben. Dieses Verschieben dient dem Einstechen einer Injektionsnadel N im Zuge einer Autoinjektion.

Das Behältnis B ist kreiszylinderisch mit einer seitlichen Aufweitung im Bereich eines vorderen Kolbens K1. Ein Auslaß des Behältnisses B am vorderen, in der Figur linken Ende des Behältnisses B ist durch eine Membran verschlossen. Die Membran ist vor der Verwendung des Behältnisses B von der Injektionsnadel N durchstochen worden. In dem Behältnis B sind hintereinander zwei Kolben K1 und K2 verschiebbar aufgenommen. Im Ausgangszustand befinden sich ein in Pulverform vorliegender Wirkstoff in einer vorderen, in der Figur linken Behältniskammer und eine Trägerflüssigkeit in einer zwischen den beiden Kolben K1 und K2 gebildeten hinteren Behältniskammer.

Das injizierbare Produkt, die Wirkstofflösung, wird durch Vorschieben des hinteren Kolbens K2 gegen den vorderen Kolben K1 gebildet. Die Trägerflüssigkeit wird dabei über die seitliche Aufweitung der Behältniswandung in die vordere Kammer verdrängt. Der Wirkstoff ist dann in der Trägerflüssigkeit gelöst. Dieser Zustand ist in Fig. 1 dargestellt.

Das Vorschieben des hinteren Kolbens K2 wird beim Zusammensetzen der vorderen Gehäusehülse 2 und der hinteren Gehäusehülse 3 bewirkt. Zu diesem Zweck ist ein als Hülsenkörper ausgebildetes Mischglied 10 in der hinteren Gehäusehülse 3 verdrehgesichert aufgenommen. Das Mischglied 10 weist einen vorderen Hülsenbereich mit einem Außendurchmesser, der kleiner als der Innendurchmesser des Behältnisses B ist, und einen gegenüber diesem vorderen Hülsenbereich verbreiterten hinteren Hülsenbereich auf. Ein Übergang zwischen diesen beiden Hülsenbereichen ist als radial vom vorderen Hülsenbereich abragende Schulter 14 ausgebildet. Die Schulter 14 ist umlaufend ausgebildet; sie kann jedoch auch durch wenigstens einen radial abragenden Steg gebildet werden. Mit seiner hinteren Stirnfläche stößt das Mischglied 10 gegen radial von der hinteren Gehäusehülse 3 nach innen ragende Stege 6, die auch als umlaufende Wandung ausgebildet sein können, an. Beim Zusammensetzen des Geräts, d.h. beim Verschrauben der beiden Gehäusehülsen 2 und 3, wird das derart verschiebegesichert in der hinteren Gehäusehülse 3 aufgenommene Mischglied 10 in das rückwärtig offene Behältnis B eingeführt und darin nach vorn geschoben. Dabei drückt das Mischglied 10 den hinteren Kolben K2 nach vorn auf den vorderen Kolben K1 zu bis der hintere Kolben K2 die in Fig. 1 gezeigte Stellung erreicht hat. In dieser Stellung der Kolben K1 und K2 ist auch das Verschrauben der Gehäusehülse 2 und 3 abgeschlossen.

In der hinteren Gehäusehülse 3 ist eine Antriebseinrichtung angeordnet, die im Ausführungsbeispiel ein Antriebselement 5 in Form einer Druckfeder und ein stangenförmiges Abtriebsglied 20 umfaßt, das im Gehäuse geradgeführt wird. Das Antriebselement 5 ist zwischen den Stegen 6 und einer den Stegen 6 in Vorschubrichtung gegenüberliegend zugewandten, umlaufenden Schulterfläche des Abtriebsglieds 20 eingespannt.

Das Abtriebsglied 20 ist um eine Mittellängsachse des Gehäuses, die mit seiner eigenen Mittellängsachse zusammenfällt, zwischen zwei Drehstellungen hin- und her verdrehbar aufgenommen. Zum Verdrehen des Abtriebsglieds 20 ist eine das Gehäuse verlängernde Dosierhülse D vorgesehen. In seinem rückwärtigen Bereich, der in die Dosierhülse D hineinragt, weist das Abtriebsglied 20 in Vorschubrichtung sich erstreckende Führungsnuten 21 auf, in die eine in die Abdeckkappe 4 hineinragende, der Abdeckkappe 4 gegenüber verdrehbare, verschiebegesicherte Führungshülse 28 und eine im Gehäuse verdrehbar und verschiebegesichert aufgenommene Anzeigehülse 8 eingreifen. Die Führungshülse 28 ist mit der Dosierhülse D verdrehgesichert verbunden, wie am besten im Schnitt H-H zu erkennen ist. Die Führungshülse 28 dient der Übertragung der Drehbewegung der Dosierhülse D auf das Abtriebsglied 20. Die Anzeigehülse 8, die verdrehgesichert mit dem Abtriebsglied 20 verbunden ist, dient der Anzeige der Verdrehstellung des Abtriebsglieds 20 und damit der Anzeige der eingestellten Dosismenge. Sie ist zu diesem Zweck an ihrem äußeren Umfang mit Markierungen versehen, im Ausführungsbeispiel mit zwei Markierungen für je eine von zwei Drehendstellungen des Abtriebsglieds 20. Die Markierungen können durch eine Gehäuseöffnung hindurch abgelesen werden. Die Anzeigehülse 8 und auch die Führungshülse 28 dienen zusammen mit dem Mischglied 10 als Geradführung für das Abtriebsglied 20.

Das Abtriebsglied 20 wird in der dargestellten hinteren Ausgangsstellung durch eine Sperr- und Auslösemechanik gehalten. Die Sperr- und Auslösmechanik weist ein Auslösemittel 7a in Form eines Auslöseknopfs auf, der quer zur Verschieberichtung des Abtriebsglieds 20 auf ein Sperrmittel 7b wirkt. Der Aufbau und die Wirkungsweise der Sperr- und Auslösemechanik ist am besten in der Zusammenschau des Längsschnitts und des Querschnitts F-F zu erkennen.

Das Sperrmittel 7b wird durch einen Hülsenkörper gebildet mit einer Durchgangsöffnung, die von dem Abtriebsglied 20 durchragt wird. Das Sperrmittel 7b wird zum Zwecke seiner Geradführung quer zur Vorschub- und Längsrichtung des Abtriebsglieds 20 zwischen zwei geraden Stegen des Gehäuses geführt. Diesen beiden Gehäusestegen jeweils zugewandt weist der Hülsenkörper des Sperrmittels 7b dementsprechend gerade Außenflächen auf. Die Durchgangsöffnung des Sperrmittels 7b ist größer als der Außendurch messer des durchragenden Abtriebsglieds 20. Durch Druck auf das Auslösemittel 7a wird das Sperrmittel 7b gegen die Kraft eines als Druckfeder ausgebildeten Rückstellelements 7c quer zur Vorschubrichtung des Abtriebsglieds 20 verschoben. In der Sperrstellung stößt das Abtriebsglied 20 mit einer durch eine Verdickung gebildete Schulter 26 gegen eine hintere Stirnfläche des Sperrmittels 7b. Durch eine Querverlagerung des Spermittels 7b wird dieser Anschlag gelöst. Das Abtriebsglied 20 kommt von dem Sperrmittel 7b frei und kann unter dem Andruck des Antriebselements 5 in Längsrichtung vorschieben.

Eine Sicherheitseinrichtung sorgt dafür, daß das Auslösemittel 7a nur dann betätigt und dadurch das Abtriebsglied 20 freigegeben werden kann, wenn ein Behältnis B in das Gehäuse eingelegt worden ist. Die Sicherheitseinrichtung umfaßt einen Auslösesperrkörper 18 und eine Druckfeder 19. Der Auslösesperrkörper 18 weist einen mittleren Hülsenbereich auf, von dem nach vorne in Längsrichtung zwei Stege 18a abragen (Schnitt E-E). Die beiden Stege 18a durchragen zwei entsprechend geformte Schlitze in der Schulter 14 des Mischglieds 10 und stoßen an den hinteren Rand des Behältnisses B an. Vom mittleren Hülsenbereich des Auslösesperrkörpers 18 ragt ein dritter Steg 18b in Längsrichtung nach hinten ab. Dieser dritte Steg 18c durchragt das Auslösemittel 7a, wie am besten in der Zusammenschau des Längsschnitts mit den beiden Schnitten F-F und G-G zu erkennen ist. Auf der Höhe des Auslösemittels 7a, d.h. in dem das Auslösemittel 7a durchragenden Bereich, weist der dritte Steg 18b des Auslösesperrkörpers 18 einen Längsschlitz auf. In diesen Längsschlitz fährt eine radial nach innen ragende Rippe 7d des Auslösemittels 7a bei Betätigung des Auslösemittels 7a ein, wenn der Schlitz des Auslösesperrkörpers 18 auf gleicher Höhe wie die Innenrippe 7d des Auslösemittels 7a ist. In Längsrichtung gesehen hinter dem geschlitzten Bereich ist der dritte Steg 18b des Auslösesperrkörpers 18 wieder als geschlossener Steg augebildet. Zwischen dem gehäuseseitigen Steg 6 und einer von der inneren Mantelfläche des mittleren Hülsenbereichs des Auslösesperrkörpers 18 nach innen vorragenden Schulter ist die Druckfeder 19 gespannt. Ist ein Behältnis B eingesetzt, so drückt der Auslösesperrkörper 18 mit seinen beiden vorderen Stegen 18a gegen den hinteren Rand des Behältnisses B und wird so in der im Längsschnitt der Figur 1 dargestellten Stellung gehalten, in der die Innenrippe 7d des Auslösemittels 7a in den Schlitz des Auslösesperrkörpers 18 einfahren und das Sperrmittel 7b querverschieben kann. Falls ein Behältnis nicht eingesetzt worden ist, wird der Auslösesperrkörper 18 durch die Druckfeder 19 in den deshalb dann freien Ringspalt vorgeschoben, bis der mittlere Hülsenbereich des Auslösesperrkörpers 18 gegen die Schulter 14 des Mischglieds 10 stößt. In dieser Sperrstellung des Auslösersperrkörpers 18 kommt der hintere, geschlossene Bereich des dritten Steges 18b des Auslösesperrkörpers 18 vor die Innenrippe 7d des Auslösemittels 7a zu liegen. Ein Querverschieben des Auslösemittels 7a ist diesem Fall nicht möglich. Die Antriebseinrichtung ist dann gesperrt.

Zwischen dem Mischglied 10 und dem das Mischglied 10 durchragenden Abtriebsglied 20 besteht eine Antriebskopplung, die bewirkt, daß das Mischglied 10 bei einem Vorschieben des Abtriebsglieds 20 vom Abtriebsglied 20 mitgenommen, d.h. selbst relativ zum Gehäuse vorgeschoben wird. Die Kopplung wird durch eine auf Form- und Kraftschluß beruhende Verbindung im vorderen Bereich des Mischglieds 10 hergestellt. Die Kopplung wird durch ein erstes Kopplungsmittel 13, ein zweites Kopplungsmittel 24 und ein drittes Kopplungsmittel 25 gebildet.

Das erste Kopplungsmittel 13 ist ein vorderer Steg einer zwischen zwei an der Innenmantelfläche des Mischglieds 10 umlaufenden Stegen ausgebildeten Führungsnut für das dritte Kopplungsmittel 25. Das dritte Kopplungsmittel 25 ist ein nachgiebiger Ring, im Ausführungsbeispiel ein federelastischer Ring, der längs in der Art eines Kolbenrings einmal geschlitzt ist. Das zweite Kopplungsmittel 24 wird durch eine Schulter gebildet, die durch Verbreiterung des stangenförmigen Abtriebsglieds 20 entsteht. Mit diesem zweiten Kopplungsmittel 24 drückt das Abtriebsglied 20 bei seinem Vorschieben gegen das dritte Kopplungsmittel 25 und dieses gegen das erste Kopplungsmittel 13, so daß ein Vorschieben des Abtriebsglieds 20 auch ein Vorschieben des Mischglieds 10 bewirkt. Das Mischglied 10 dient gleichzeitig als Übertragungsglied, das mit seiner umlaufenden Schulter 14 gegen den Behältnishalter 30 und das Behältnis B drückend die Vorschubbewegung des Abtriebsglieds 20 auf den Behältnishalter 30 und das Behältnis B überträgt. Die Schulter 14 erfüllt die Funktion eines Mitnehmers für das Behältnis B.

Das Mischglied 10 und das Abtriebsglied 20 bilden in einem innerhalb des Behältnisses B angeordneten Bereich eine Dosiereinrichtung. Das Mischglied 10 ist hierfür in einem hinter dem ersten Kopplungsmittel 13 befindlichen Innenmantelbereich mit einer Ausnehmung versehen. Die Ausnehmung weist zwei sich in Vorschubrichtung erstreckende Nuten 11 und 12 auf, die winkelversetzt parallel nebeneinander angeordnet sind. Die Nuten 11 und 12 sind in Vorschubrichtung unterschiedlich lang. Die kürzere Nut 11 ist als Sacknut in der Mantelfläche ausgebildet, und die längere Nut 12 wird in Vorschubrichtung durch den hinteren Steg der Aufnahme für das dritte Kopplungsmittel 25 begrenzt. An Ihren rückwärtigen Enden münden die Nuten 11 und 12 auf gleicher Höhe in Bezug auf die Vorschubrichtung in einer Verbreiterung der Ausnehmung aus, wie am besten in Zusammenschau des Längsschnitts mit den Schnitten C-C, D-D und E-E zu erkennen ist. Die Verbreiterung der Ausnehmung reicht bis zur hinteren Stirnfläche des vorderen Hülsenbereichs des Mischglieds 10. Die dort auslaufenden, einander gegenüberliegend zugewandten Seitenwände der Verbreiterung werden je durch eine der beiden Nuten 11 und 12 in Vorschubrichtung verlängert.

Das stangenförmige Abtriebsglied 20 ist mit einem radial abragenden Nocken 23 versehen. Im Ausgangszustand des Injektionsgeräts greift der Nocken 23 in die Verbreiterung der Ausnehmung des Mischglieds 10 ein. Die Ausnehmung mit den beiden Nuten 11 und 12 bildet ein erstes Dosiermittel und der Nocken 23 ein zweites Dosiermittel der Dosiereinrichtung.

In einer ersten Dosierstellung liegt der Nocken 23 in Flucht zur Nut 11 an der in Vorschubrichtung sich erstreckenden ersten Seitenwand der Verbreiterung an, und in der zweiten Dosierstellung liegt der Nocken 23 fluchtend zur Nut 12 an der in Vorschubrichtung sich erstreckenden zweiten Seitenwand der Verbreiterung der Ausnehmung an. Zwischen diesen beiden Dosierstellungen ist das Abtriebsglied 20 im Ausgangszustand des Injektionsgeräts hin- und her um seine Längsachse verdrehbar. Die beiden Seitenwände der breiten Nut definieren die beiden Dreh- und Dosierstellungen des Abtriebsglieds 20, und die Längen der beiden schmalen Nuten 11 und 12 definieren die Menge der bei einer Injektion ausschüttbaren Wirkstofflösung.

Die Verbreiterung der Ausnehmung im Mischglied 10 könnte auch bis zum Ende der kurzen Nut 11 in Vorschubrichtung verlängert werden, so daß die Ausnehmung in Vorschubrich-tung einen einfach treppenförmigen Verlauf aufweisen würde.

Zum Durchführen einer Autoinjektion wird das Autoinjektionsgerät mit einer vorderen, dem Gehäuse bzw. dem vorderen Nadelschutz 1 gegenüber zurückschiebbaren Nadelschutzhülse 9 auf eine Gewebeoberfläche, insbesondere die menschliche Haut, aufgesetzt. Durch Druck gegen die Gewebeoberfläche wird die Nadelschutzhülse 9 bis in eine hinterste Position relativ zum Gehäuse zurückgeschoben. Die Injektionsnadel, die an einem Auslaß am vorderen Ende des Behältnisses B in Vorschubrichtung weisend fest angebracht ist, wird zunächst noch von dem Nadelschutz 1 und der darübergeschobenen Nadelschutzhülse 9 bis über ihre vordere Spitze hinaus umgeben und berührt somit noch nicht die Gewebeoberfläche.

Zur Betätigung, d.h. zum Einstechen der Nadel und zur Ausschüttung der Wirkstofflösung, drückt der Verwender, nachdem er das Abtriebsglied 20 mittels der Dosierhülse D in die gewünschte Dreh- bzw. Dosierstellung gebracht hat, das Auslösemittel 7a in radialer Richtung einwärts. Durch das Einwärtsdrücken wird das Sperrmittel 7b unter der Anschlagschulter 26 weggeschoben, und das Abtriebsglied 20 kommt frei. Unter dem Andruck des Antriebselements 5 wird das Abtriebsglied 20 und mittels der Kopplung auch das Mischglied 10 relativ zum Gehäuse vorgeschoben. Der Form- und Kraftschluß zwischen dem Abtriebsglied 20 und dem Mischglied 10 ist ausreichend stark, um die Mitnahme des Mischglieds 10 zu bewirken, das seinerseits mittels seiner Schulter 14 gegen den Behältnishalter 30 und das Behältnis B drückend diese relativ zum Gehäuse und entgegen der rückstellenden Kraft des Rückstellements 31 bis in eine vordere Position vorschiebt, die durch einen gehäuseseitigen Anschlag 32 definiert wird.

In der vorderen Position des Behältnishalters 30 bzw. des Behältnisses B löst die Kopplung die Antriebsverbindung zwischen dem Abtriebsglied 20 und dem Mischglied 10. Unter dem weiterhin bestehenden Andruck des Antriebselements 5 wird wegen der Fixierung des Mischglieds 10 der nachgiebige Ring 25 zusammengedrückt und über die schräge Schulter 24 geschoben. Das Abtriebsglied 20 schiebt nun auch relativ zum Mischglied 10 weiter vor und drückt dabei die beiden Kolben K1 und K2 im Behältnis in Richtung auf den Behältnisauslaß zu ebenfalls vor. Wirkstofflösung wird durch die in der vorderen Position des Behältnisses B in das Gewebe eingestochene Nadel hindurch ausgeschüttet.

Die Vorschubbewegung des Abtriebsglieds 20 wird in der ersten Dosierstellung durch das vordere Ende der Nut 11 begrenzt. In der ersten Dosierstellung ist die Ausschüttung beendet, wenn der Nocken 23 an diese in Umfangsrichtung sich erstreckende Nutwand anstößt.

Nach dem Herausziehen der Nadel N wird das Injektionsgerät für eine zweite Injektion bereitgemacht. Hierfür ist lediglich das Abtriebsglied 20 zunächst dem Mischglied 10 gegenüber entgegen der Vorschubrichtung zurückzuziehen. Am vorderen Ende weist das Abtriebsglied 20 einen Stempel 22 in Form einer flanschartigen Verbreiterung auf. Beim Ausschütten der Wirkstofflösung drückt das Abtriebsglied 20 mit dem Stempel 22 auf den hinteren Kolben K2, und beim Zurückziehen stößt die rückwärtige, umlaufende Schulterfläche des Stempels 22 gegen den von der Innenmantelfläche des Mischglieds 10 vorstehenden Steg 13. Beim weiteren Zurückziehen des Abtriebsglieds 20 wird hierdurch das Mischglied 10 mitgenommen, d.h. ebenfalls zurückverschoben, bis in seine in Fig. 1 gezeigte hintere Stellung. Der Behältnishalter 30 und das darin aufgenommene Behältnis B folgen dabei unter dem Andruck des Rückstellelements 31 der Bewegung des Mischglieds 10. Die Rückstellkraft des Rückstellelements 31 ist gegenüber der Antriebskraft des Antriebselements 5 vergleichsweise gering, so daß es beim Vorschieben des Behältnisses B zum Zwecke des Einstechens der Nadel N nicht stört.

Für die nächste Injektion wird das Abtriebsglied 20 in seine zweite Dosierstellung gedreht, in der der Nocken 23 in der Flucht zur Nut 12 steht. In dieser Stellung kann das Abtriebsglied 20 relativ zum Mischglied 10 so weit vorgeschoben werden, daß die noch im Behältnis verbliebene Restmenge der Wirkstofflösung bei einer Betätigung der Antriebseinrichtung, d.h. einer Betätigung des Auslösemittels 7, ausgeschüttet wird. Die Vorschubbewegung wird durch einen Anschlagflansch 27 begrenzt.

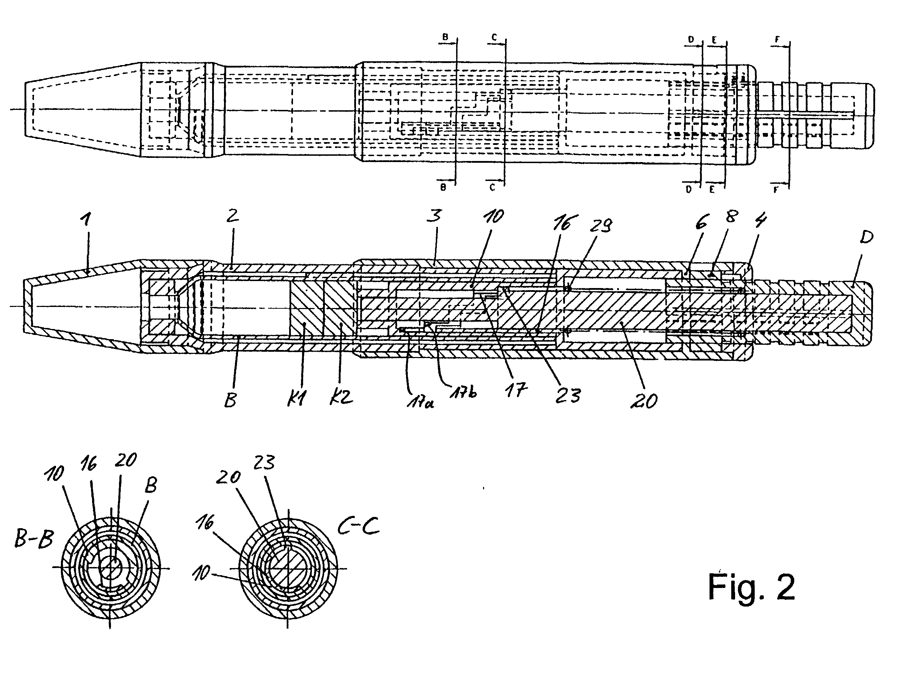

Fig. 2 zeigt ein Injektionsgerät mit einer im Behältnis B angeordneten Dosiereinrichtung, die es ermöglicht, vier vorgegebene Dosismengen Wirkstofflösung auszuschütten. Das Injektionsgerät ist ein einfaches Injektionsgerät in dem Sinne, daß der Verwender die Nadel manuell einsticht und auch die Ausschüttung durch beständigen manuellen Druck auf den Dosierknopf D des Abtriebsglieds 20 bewirkt.

Das Mischglied 10 erfüllt im Ausführungsbeispiel der Fig. 2 die beiden Funktionen des Mischens der Wirkstofflösung und der Dosierung der auszuschüttenden Menge der Wirkstofflösung. Im folgenden werden insbesondere Unterschiede zum Injektionsgerät der Fig. 1 beschrieben. Bezüglich der offensichtlichen Gemeinsamkeiten wird auf die dortige Beschreibung verwiesen. Bauteile gleicher Funktion sind dementsprechend auch mit den Bezugszeichen des Ausführungsbeispiels der Fig. 1 versehen.

Das Behältnis B ist in der vorderen Gehäusehülse 2 verschiebegesichert aufgenommen.

Das Mischglied 10 ist nach dem Zusammenschrauben der beiden Gehäusehülsen 2 und 3, d.h. nach Ausübung seiner Mischfunktion, im Gehäuse ebenfalls verschiebegesichert aufgenommen. Es weist in Vorschubrichtung des Abtriebsglieds 20 gesehen einen vorderen Hülsenabschnitt, einen demgegenüber breiteren mittleren Hülsenabschnitt und einen nochmals verbreiterten hinteren Hülsenabschnitt auf, der eng umhüllend im Gehäuse sitzt. Der vordere Hülsenabschnitt des Mischglieds 10 dient als Gleitführung für das Abtriebsglied 20. Der mittlere Hülsenabschnitt reicht bis zur Behältnisinnenwandung.

Im mittleren Hülsenabschnitt weist das Mischglied 10 an einer inneren Mantelfläche einen treppenförmigen Führungskanal 17 auf. Dabei wird jeweils ein hinterer, in Vorschubrichtung sich erstreckender Kanalabschnitt durch einen daran anschließenden, in Umfangsrichtung sich erstreckenden Kanalabschnitt mit einem in Vorschubrichtung gesehen nächst vorderen, wieder in Vorschubrichtung weisenden Kanalabschnitt verbunden. Der Führungskanal 17 bildet das erste Dosiermittel. Das zweite Dosiermittel 23 ist wieder als Nocken ausgebildet, der unmittelbar von einer äußeren Mantelfläche des Abtriebsglieds 20 abragt und im Führungskanal 17 beim Vorschieben des Abtriebsglieds 20 geführt wird. Durch den Verlauf des Führungskanals 17 mit der in Vorschubrichtung gesehen beidseitigen Führung des Nockens 23 wird ein versehentliches Fehldosieren sicher verhindert. Ein Drehen in die nächste Dosierstellung ist. erst möglich, wenn der Nocken 23 das Ende eines in Vorschubrichtung sich erstreckenden Kanalabschnitts erreicht hat, d.h. wenn die vorgegebene Produktdosis der vorhergehenden Dosierstellung ausgeschüttet worden ist.

Die Dosierstellung des Abtriebsglieds 20 relativ zum Gehäuse kann wieder an einer Anzeigehülse 8, die verdrehgesichert an dem Dosierknopf D befestigt ist, durch eine Gehäuseöffnung hindurch abgelesen werden. Der Dosierknopf D ist auf den hinteren Bereich des Abtriebsglieds aus dem Gehäuse nach hinten vorragend aufgesteckt. Er bildet in seinem vorderen Bereich einen Ringspalt um das Abtriebsglied 20. In diesem Ringspalt ist ein als Druckfeder wirkendes Rückstellelement 29 aufgenommen, das gegenüberliegend im Schulterbereich zwischen dem hinteren und dem mittleren Hülsenabschnitt des Mischglieds 10 abgestützt ist.

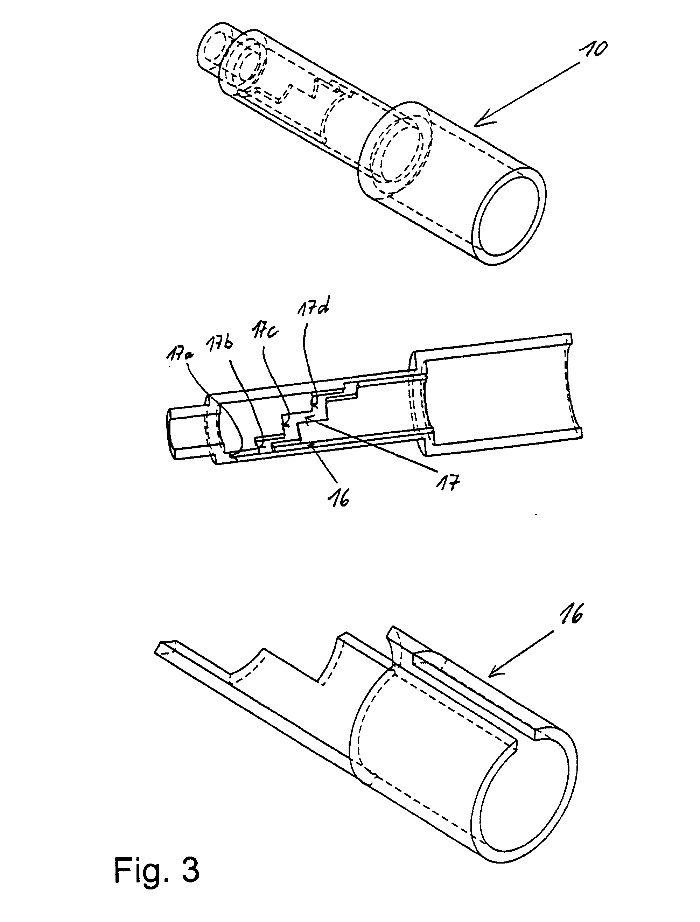

Das Mischglied 10 ist in Figur 3 einzeln dargestellt. Das erste Dosiermittel, nämlich der am Innenmantelbereich des Mischglieds 10 ausgebildete Führungskanal 17 wird durch Zusammenfügen mehrerer Komponenten erhalten. Der mittlere Hülsenabschnitt des Mischglieds 10 ist im Ausführungsbeispiel einfach kreiszylindrisch. Zur Ausbildung des Führungskanals 17 ist in diesen Hülsenabschnitt ein zusätzlicher Hülsenkörper 16 eingeschoben und in seiner Winkellage geeignet befestigt. Der mittlere Hülsenabschnitt des Mischglieds 10 weist einen nach innen erhaben vorstehenden Bereich mit einer rückwärtigen, treppenförmig verlaufenden Stirnfläche auf. Der zusätzliche Hülsenkörper 16 weist eine diesem Treppenverlauf folgende Kontur auf, mit der er dem erhabenen Bereich des mittleren Hülsenabschnitts beabstandet gegenüberliegend in das Mischglied 10 eingeschoben ist. Die eineinander zugewandten Stirnflächen des mittleren Hülsenabschnitts und des Hülsenkörpers 16 bilden die Seitenwände des Führungskanals 17, insbesondere werden so gegen die Vorschubrichtung weisende Anschlagflächen 17a, 17b, 17c und 17d gebildet. Die Höhen, auf denen diese Anschlagflächen 17a bis 17d liegen, bestimmen die Dosismengen bei einer Ausschüttung der Wirkstofflösung. Der erhabene Bereich im Mischglied 10 kann auch durch einen separaten Schalen- oder Hülsenkörper gebildet werden, der ebenfalls im dann einfach glatten Hülsenkörper des Mischglieds 10 zu befestigen ist.

Die Ausbildung der jeweils ersten Dosiermittel kann in beiden Ausführungsbeispielen vertauscht werden.

## Patentansprüche

1. Vorrichtung zur dosierten Verabreichung eines injizierbaren Produkts, wenigstens umfassend:
a) ein Basisteil (1, 2, 3),
b) ein vom Basisteil (1, 2, 3) aufgenommenes Behältnis (B), aus dem durch Vorschieben wenigstens eines darin aufgenommenen Kolbens (K1, K2) in eine Vorschubrichtung eine Produktdosis durch eine Nadel hindurch ausgeschüttet wird,
c) eine Antriebseinrichtung wenigstens mit einem in das Behältnis (B) ragenden Abtriebsglied (20), das bei Betätigung der Antriebseinrichtung den Kolben (K1, K2) in Vorschubrichtung schiebt, und
d) eine Dosiereinrichtung zur Einstellung derjenigen Weglänge, die das Abtriebsglied (20) bei einer Betätigung der Antriebseinrichtung relativ zum Basisteil (1, 2, 3) in Vorschubrichtung des Kolbens (K1, K2) verschoben wird,
**dadurch gekennzeichnet, daß**
e) wenigstens ein erstes Dosiermittel (11, 12; 17) und wenigstens ein relativ dazu verstellbares zweites Dosiermittel (23) der Dosiereinrichtung in dem Behältnis (B) angeordnet sind und die Dosierung im Zusammenwirken des ersten und des zweiten Dosiermittels innerhalb des Behältnisses (B) stattfindet.

2. Vorrichtung nach Anspruch 1, **dadurch gekennzeichnet, daß** das zweite Dosiermittel (23) bei einem Vorschieben des Abtriebsglieds (20) an einen Anschlag anstößt und dadurch die Verschiebeweglänge begrenzt wird.

3. Vorrichtung nach Anspruch 1 oder 2, **dadurch gekennzeichnet, daß** das zweite Dosiermittel (23) unmittelbar mit dem Abtriebsglied (20) verbunden ist.

4. Vorrichtung nach wenigstens einem der vorhergehenden Ansprüche, **dadurch gekennzeichnet, daß** das erste Dosiermittel (11, 12; 17) durch eine Ausnehmung in einer Zylindermantelfläche gebildet wird, wobei die Ausnehmung wenigstens zwei in Vorschubrichtung sich erstreckende Seitenwände aufweist und eine Verbindung dieser Seitenwände einen in Umfangsrichtung sich erstreckenden und gegen die Vorschubrichtung weisenden, als Anschlag für das zweite Dosiermittel (23) dienenden Wandabschnitt umfaßt.

5. Vorrichtung nach dem vorhergehenden Anspruch, **dadurch gekennzeichnet, daß** das erste Dosiermittel (11, 12; 17) ein Führungskanal für das zweite Dosiermittel (23) ist.

6. Vorrichtung nach dem vorhergehenden Anspruch, **dadurch gekennzeichnet, daß** der Führungskanal durch Zusammenfügen von zwei zylindrischen Körpern (10, 16) erhalten wird, wobei zumindest einer (16) dieser Körper ein Hülsenkörper ist, der eine Stirnfläche aufweist, die eine Seitenwand des Führungskanals bildet.

7. Vorrichtung nach wenigstens einem der vorhergehenden Ansprüche 1, **dadurch gekennzeichnet, daß** das Behältnis (B) eine Mehrkammerampulle mit wenigstens zwei in Vorschubrichtung hintereinander angeordneten Kammern ist, die durch einen vorderen Kolben (K1) voneinander getrennt und durch einen hinteren Kolben (K2) abgeschlossen werden, der bei einem Vermischen der Inhalte der beiden Kammern mittels eines in das Behältnis (B) hineinragenden, im Behältnis (B) vorschiebbaren Mischglieds (10) auf den vorderen Kolben (K1) zu verschoben wird, und daß das Mischglied (10) das erste Dosiermittel (11, 12; 17) aufweist.

8. Autoinjektionsgerät, **dadurch gekennzeichnet, daß** das Gerät eine Vorrichtung nach wenigstens einem der vorhergehenden Ansprüche umfaßt, wobei das Behältns (B) vom Abtriebsglied (20) zum Einstechen der am Behältnis (B) befestigten Nadel relativ zum Basisteil (1, 2, 3) vorgeschoben wird.

9. Autoinjektionsgerät nach dem vorhergehenden Anspruch, **dadurch gekennzeichnet, daß** eine das erste Dosiermittel (11, 12) aufweisende Komponente (10) der Dosiereinrichtung als Übertragungsglied dienend eine Vorschubbewegung des Abtriebsglieds (20) auf das Behältnis (B) überträgt, bis das Behältnis (B) relativ zum Basisteil (1, 2, 3) eine vordere Position erreicht hat.

## Claims

1. Device for the dosed administration of an injectable product, comprising at least:
a) a base part (1, 2, 3),
b) a container (B) which is accommodated by the base part (1, 2, 3) and from which, through pushing forward in a feed direction at least one piston (K1, K2) accommodated therein, a dose of the product is dispensed through a needle,
c) a drive device at least with one output element (20) which projects into the container (B), and which pushes the piston (K1, K2) in the forward feed direction when the drive device is actuated, and
d) a dosing device for setting that path length by which the output element (20) is displaced relative to the base part (1, 2, 3) in the forward feed direction of the piston (K1, K2) when the drive device is actuated,
**characterised in that**
e) at least one first dosing element (11, 12; 17) of the dosing device and at least one second dosing element (23) that can be adjusted relative to it [the first element] are arranged in the container (B), and the dosing takes place in the interaction of the first and second dosing elements within the container (B).

2. Device in accordance with claim 1, **characterised in that** when the output element (20) is pushed forward, the second dosing element (23) hits a limit stop and thus the length of the displacement path is limited.

3. Device in accordance with claim 1 or 2, **characterised in that** the second dosing element (23) is connected directly to the output element (20).

4. Device in accordance with at least one of the preceding claims, **characterised in that** the first dosing element (11, 12; 17) is formed by a recess in a cylinder casing surface, wherein the recess has at least two side walls extending in the forward feed direction and a connection of these side walls includes a wall section which extends in the circumferential direction and points against the forward feed direction, and serves as a limit stop for the second dosing element (23).

5. Device in accordance with the preceding claim, **characterised in that** the first dosing element (11, 12; 17) is a guide channel for the second dosing element (23).

6. Device in accordance with the preceding claim, **characterised in that** the guide channel is obtained by joining together two cylindrical bodies (10, 16), wherein at least one (16) of these bodies is a sleeve body which has a frontal face that forms a side wall of the guide channel.

7. Device in accordance with at least one of the preceding claims 1, **characterised in that** the container (B) is a multi-chamber ampoule with at least two chambers which are arranged one behind the other in the forward feed direction, and which are separated from one another by a front piston (K1) and are concluded by a rear piston (K2) which, when the contents of the two chambers are mixed by means of a mixing element (10) which projects into the container (B) and can be pushed forward in the container (B), is pushed towards the front piston (K1), and that the mixing element (10) has the first dosing element (11, 12; 17).

8. Auto-injection appliance, **characterised in that** the appliance comprises a device in accordance with at least one of the preceding claims, wherein the container (B) is pushed forward relative to the base part (1, 2, 3) by the output element (20), for insertion of the needle fastened to the container (B).

9. Auto-injection appliance in accordance with the preceding claim, **characterised in that** a component (10) of the dosing device, which [component] has the first dosing element (11, 12), serving as a transmission element, transfers a forward feed movement of the output element (20) to the container (B), until the container (B) has reached a front positional relative to the base part (1, 2, 3).

## Revendications

1. Dispositif pour l'administration dosée d'un produit injectable, comprenant au moins :
a) une partie de base (1, 2, 3),
b) un récipient (B) réceptionné par la partie de base (1, 2, 3), à partir duquel, par l'avancement d'au moins un piston (K1, K2) réceptionné à l'intérieur, une dose de produit est versée en traversant une aiguille dans un sens d'avancement,
c) un système d'entraînement équipé d'au moins un élément de sortie (20) débordant dans le récipient (B), qui pousse le piston (K1, K2) dans le sens d'avancement lors de l'actionnement du système d'entraînement, et
d) un système de dosage pour le réglage de la longueur de course par laquelle l'élément de sortie (20) est déplacé lors d'un actionnement du système d'entraînement par rapport à la partie de base (1, 2, 3) dans le sens d'avancement du piston (K1, K2),
**caractérisé en ce que**
e) au moins un premier moyen de dosage (11, 12 ; 17) et au moins un second moyen de dosage (23), déplaçable par rapport au premier, du système de dosage sont disposés dans le récipient (B) et le dosage a lieu à l'intérieur du récipient (B) dans l'action conjuguée des premier et second moyens de dosage.

2. Dispositif selon la revendication 1, **caractérisé en ce que** le second moyen de dosage (23) heurte une butée lors d'un avancement de l'élément de sortie (20) et la longueur de course de déplacement est de ce fait limitée.

3. Dispositif selon la revendication 1 ou 2, **caractérisé en ce que** le second moyen de dosage (23) est relié directement à l'élément de sortie (20).

4. Dispositif selon au moins l'une quelconque des revendications précédentes, **caractérisé en ce que** le premier moyen de dosage (11, 12 ; 17) est formé par un évidement dans une surface périphérique de cylindre, l'évidement présentant au moins deux parois latérales s'étendant dans le sens d'avancement et une liaison de ces parois latérales comprenant une partie de paroi s'étendant dans le sens périphérique, et dirigé vers le sens d'avancement et servant de butée pour le second moyen de dosage (23).

5. Dispositif selon la revendication précédente, **caractérisé en ce que** le premier moyen de dosage (11, 12 ; 17) est un canal de guidage pour le second moyen de dosage (23).

6. Dispositif selon la revendication précédente, **caractérisé en ce que** le canal de guidage est obtenu par l'assemblage de deux corps (10, 16) cylindriques, au moins l'un (16) de ces corps étant un corps de douille présentant une face frontale qui forme une paroi latérale du canal de guidage.

7. Dispositif selon au moins l'une quelconque des revendications 1 précédentes, **caractérisé en ce que** le récipient (B) est une ampoule à plusieurs chambres comprenant au moins deux chambres disposées l'une derrière l'autre dans le sens d'avancement, qui sont séparées l'une de l'autre par un piston (K1) avant et sont terminées par un piston (K2) arrière, qui est déplacé lors d'un mélange des contenus des deux chambres au moyen d'un élément de mélange (10) dépassant à l'intérieur du récipient (B) et pouvant avancer dans le récipient (B) en direction du piston (K1) avant, et **en ce que** l'élément de mélange (10) présente le premier moyen de dosage (11, 12 ; 17).

8. Appareil pour autoinjection, **caractérisé en ce que** l'appareil comprend un dispositif selon au moins l'une quelconque des revendications précédentes, le récipient (B) étant avancé par rapport à la partie de base (1, 2, 3) par l'élément de sortie (20) pour le piquage de l'aiguille fixée sur le récipient (B).

9. Appareil pour autoinjection selon la revendication précédente, **caractérisé en ce qu'**une composante (10), présentant le premier moyen de dosage (11, 12), du système de dosage servant d'élément de transmission transmet un mouvement d'avancement de l'élément de sortie (20) au récipient (B) jusqu'à ce que le récipient (B) ait atteint une position avant par rapport à la partie de base (1, 2, 3).
